# EUROPÄISCHE PATENTSCHRIFT

(11) **EP 1 339 733 B1**
(45) Veröffentlichungstag und Bekanntmachung des Hinweises auf die Patenterteilung: **13.08.2008**
(21) Anmeldenummer: 01993607.9
(22) Anmeldetag: 09.11.2001
(51) Int. Cl.: C07J 41/00, C07J 1/00, C07J 21/00, C07J 71/00

(54) **VERFAHREN ZUR HERSTELLUNG VON 4-(17ALPHA-METHYL-SUBSTITUIERTEN)3-OXOESTRA-4,9-DIEN-11BETA-YL)BENZALDEHYD-(1E ODER 1Z)-OXIMEN**
METHOD FOR THE PRODUCTION OF 4-(17ALPHA-METHYL SUBSTITUTED 3-OXOESTRA-4,9-DIEN-11BETA-YL)BENZALDEHYD-(1E OR 1Z)-OXIMES
PROCEDE DE PRODUCTION DE 3-OXOESTRA-4,9-DIEN-11BETA-YL)BENZALDEHYD-(1E OU 1Z)-OXIMES SUBSTITUEES PAR 4-(17ALPHA-METHYLE)

(30) Priorität: 10.11.2000 DE 10056675
(43) Veröffentlichungstag der Anmeldung: 03.09.2003
(73) Patentinhaber: Bayer Schering Pharma Aktiengesellschaft, 13353 Berlin (DE)
(72) Erfinder: SCHUBERT, Gerd, 07743 Jena (DE); RING, Sven, 07749 Jena (DE); ERHART, Bernd, 07768 Kahla (DE); MÜLLER, Gerd, 07745 Jena (DE)
(74) Vertreter: Ziebig, Marlene
(86) Internationale Anmeldenummer: PCT/DE2001/004217
(87) Internationale Veröffentlichungsnummer: WO 2002/038581

(56) Entgegenhaltungen:
- EP-A- 0 648 778
- DD-A- 289 539

## Beschreibung

Die Erfindung betrifft ein Verfahren zur Herstellung von 4-(17α-Methyl-substituierten 3-Oxoestra-4,9-dien-11β-yl)benzaldehyd-(1E oder 1Z)-oximen der allgemeinen Formel (I) 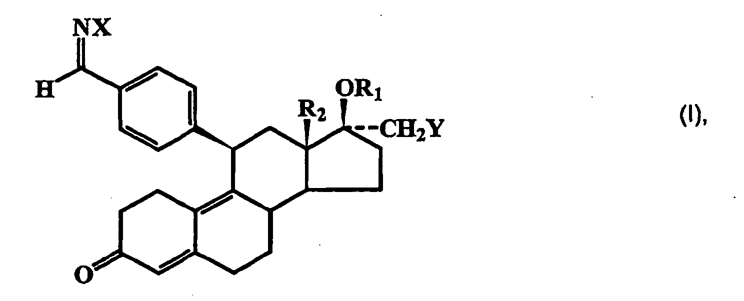 worin R₁ ein Wasserstoffatom, ein C₁₋₆-Alkylrest oder ein CₙF₂ₙ₊₁-Rest ist, wobei n 1, 2 oder 3 ist, R₂ ein C₁₋₄-Alkylrest oder eine Trifluoromethylgruppe ist, X eine E- oder Z-ständige OH-Gruppe ist und Y eine OC₁₋₆-Alkylgruppe, SC₁₋₆-Alkylgruppe oder OCH₂CₙF₂ₙ₊₁-Gruppe ist, wobei n 1, 2 oder 3 ist.

4-(17α-Methyl-substituierte 3-Oxoestra-4,9-dien-11β-yl)benzaldehyd-(1E oder 1Z)-oxime sind bereits bekannt. Substanzen dieser Art sind in DE 4332283 A1 (EP 0 648 778 B1) beschrieben. Die Verbindungen sind wegen der günstigen antigestagenen und geringen antiglucocorticoiden Wirkung von allgemeinem Interesse für die Behandlung einer Reihe von hormonabhängigen Erkrankungen der Frau, wie beispielsweise der Endometriose.

Das bisherige Verfahren zu ihrer Herstellung geht von einem am C-3 als Ketal, vorzugsweise dem als Dimethylketal geschützten 5α,10α-Epoxy-estr-9(11)-en-17-on der Formel (II) 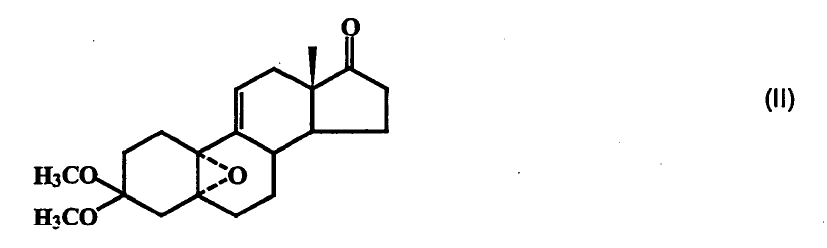 aus. In einem ersten Schritt wird in an sich bekannter Art und Weise das 5α,10α-Epoxid der Formel (II) durch eine von Cu(I)Salzen katalysierte Grignard-Reaktion mit einem 4-Brombenzaldehydketal, vorzugsweise dem 4-Brombenzaldehyddimethylketal, zu 11β-arylsubstituierten 5α-Hydroxysteroiden der Formel (III) 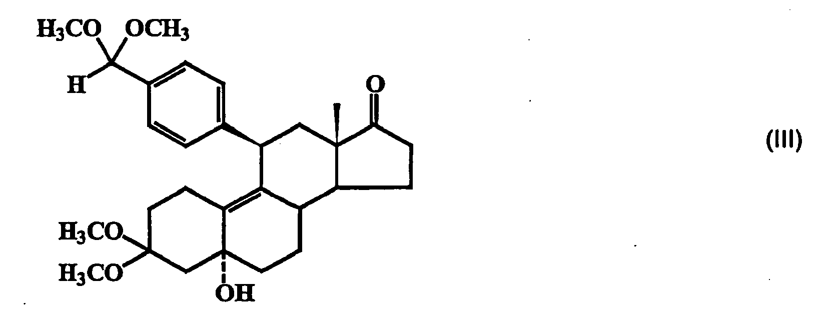 geöffnet. Die Ausbeute des Verfahrens ist dabei nicht optimal, da auch ein Teil (3 bis 10 %) der 17-Oxogruppe angegriffen wird, wobei 11β,17α-Bisaryl-substituierte Steroide der Formel (IV) 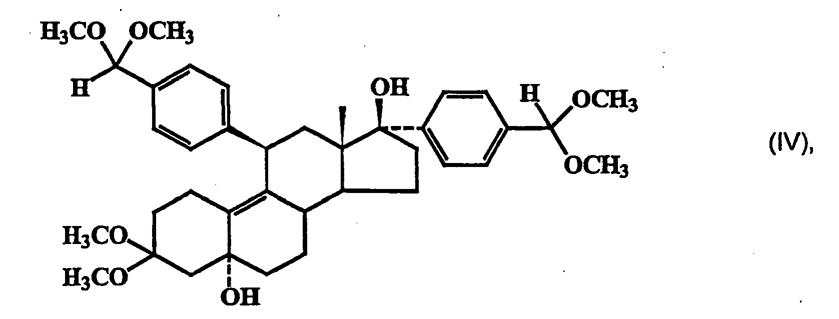 entstehen, die sich nur aufwendig durch Chromatographie von den gewünschten 11β-mono-arylsubstituierten Verbindungen der Formel (III) abtrennen lassen.

Das Gemisch der Verbindungen der Formel (III) und (IV) wird nach COREY und CHAYKOWSKY [J. Amer. Chem. Soc. 84, 3782 (1962)] hauptsächlich in das Spiroepoxid der Formel (V) 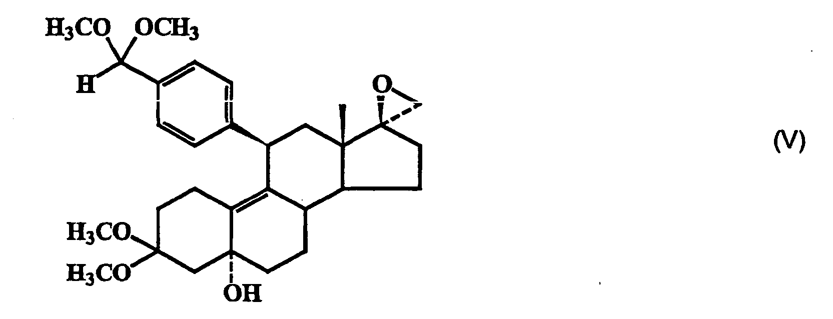 überführt, das durch Alkalimethylat zur 17α-Methoxyverbindung der Formel (VI) 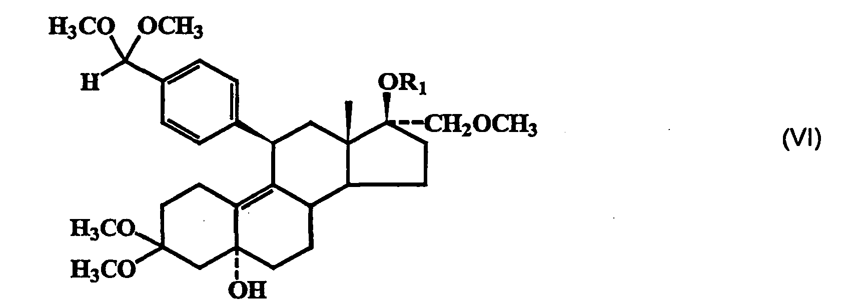 geöffnet wird, worin R₁ ein Wasserstoffatom ist. Die Verbindung der Formel (IV) wird entweder direkt oder nach Veretherung der 17β-Hydroxylgruppe mit Alkylhalogeniden in Gegenwart von Basen zu Verbindungen der allgemeinen Formel (VI), worin R₁ ein C₁₋₆-Alkylrest ist, durch saure Hydrolyse in die Benzaldehyde der Formel (VII) 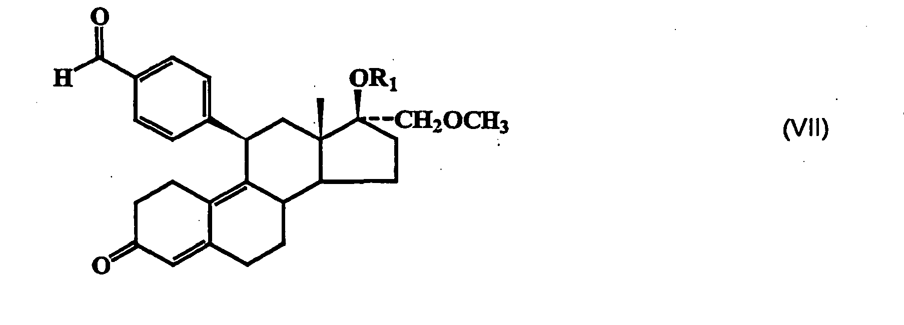 überführt, worin R₁ ein Wasserstoffatom oder ein C₁₋₆-Alkylrest ist. Die bei der Grignard-Reaktion als Nebenprodukt entstehenden 11β,17β-Bisarylsteroide der Formel (IV) werden unter den genannten Bedingungen ständig mitgeführt und letztlich zu den Bisaldehyden der Formel (VIII) 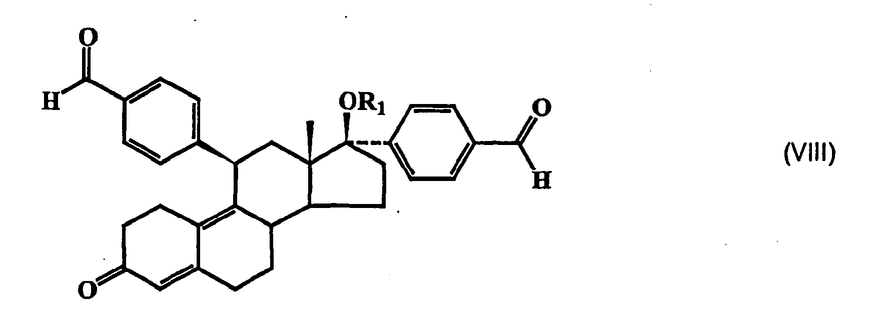 hydrolysiert. Diese Bisaldehyde der Formel (VIII) unterscheiden sich im Kristallisationsverhalten und in ihren chromatographischen Eigenschaften nur geringfügig von den Monoaldehyden der Formel (VII) und sind nur schwer quantitativ abzutrennen, stören also bei der Herstellung der erfindungsgemäßen Verbindungen der Formel (I).

Aufgabe der vorliegenden Erfindung ist deshalb ein technisch einfacheres und effektiveres Verfahren zur Herstellung von 4-(17α-Methyl-substituierten 3-Oxoestra-4,9-dien-11β-yl)benzaldehyd-(1 E oder 1Z)-oximen der Formel (1) zur Verfügung zu stellen, das den Angriff der Grignard-Verbindung am C-17 verhindert und mit höherer Ausbeute und Selektivität zu den Zielverbindungen der Formel (I) führt.

Diese Aufgabe wird gemäß dem Verfahren nach Anspruch 1 gelöst.

Dadurch, daß die 17-Ketogruppe vor der Grignardierung in die gewünschte 17α-Methyl-substituierte Verbindung überführt wird, kann die Bildung des Nebenprodukts der Formel (VIII) verhindert werden, wodurch die Zielverbindungen mit höherer Ausbeute und Reinheit erhalten werden. So kann beispielsweise ausgehend von der Verbindung (II) nach dem Verfahren gemäß DE 43 32 283 A1 der Aldehyd (VIIb) in ca. 5,6 % Ausbeute und entsprechend das Oxim (Ic) in ca. 3,8 % Ausbeute hergestellt werden. Durch das erfindungsgemäße Verfahren läßt sich der Aldehyd (VIIb) nunmehr in ca. 33 % Ausbeute bzw. das Oxim (lc) in ca 23 % Ausbeute aus dem Olefin (IX) herstellen, ohne das spezielle chromatographische Bedingungen für die Reinigung angewendet werden müssen.

Bevorzugte Ausführungsformen der Erfindung sind in den Unteransprüchen angegeben. Wegen weiterer Vorteile der Erfindung wird auf die folgende Beschreibung und die Ausführungsbeispiele verwiesen.

Erfindungsgemäß wird das 3,3-Dimethoxy-estra-5(10),9(11)-en-17-on der Formel (IX) 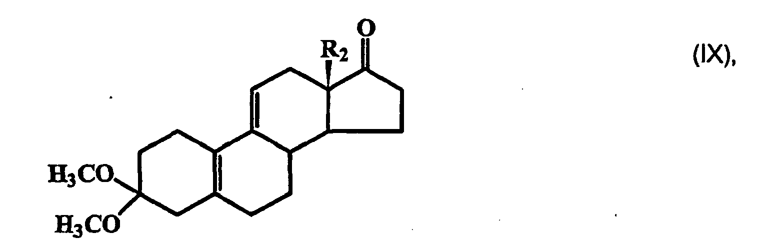 in der R₂ ein C₁₋₄-Alkylrest ist, mit einem aktiven Methylenreagenz, das z. B. aus Trimethylsulfoniumiodid und einer starken Base, wie Kalium-tert.-butanolat, in Lösungsmitteln, wie DMSO, DMF oder Toluol, hergestellt wird, in das Spiroepoxid der Formel (X) 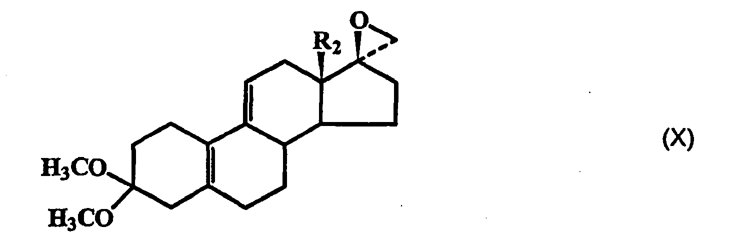 umgewandelt, in der R₂ die vorstehend gegebene Bedeutung hat, das nach Spaltung der 17-Spiroepoxygruppe durch Alkali- oder Erdalkalialkoholat, Alkali- oder Erdalkalithiolalkoholat oder durch Trifluoralkylalkohole und Kallum-tert.-butanolat, vorzugsweise durch Natriummethanolat in Lösungsmitteln, wie Methanol, DMF oder DMSO, zur 17α-CH₂Y-Verbindung der Formel (XI) 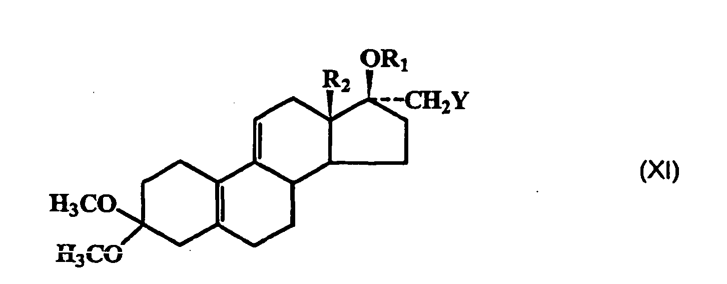 geöffnet wird, worin R₁ ein Wasserstoffatom darstellt und R₂ die vorstehend gegebene Bedeutung hat und Y eine OC₁₋₆-Alkylgruppe, SC₁₋₆-Alkylgruppe oder OCH₂CₙF₂ₙ₊₁-Gruppe ist, wobei n 1, 2 oder 3 ist.

Durch Umsetzung der 17β-Hydroxylgruppe mit Alkylhalogeniden oder Fluoralkylhalogeniden (Halogen = Chlor, Brom oder lod), wie Fluoralkyliodid, in Gegenwart von starken Basen, wie Kaliumhydroxid, Alkoholaten, wie Kalium-tert.-butanolat, Silberfluoriden, Alkalimetallen und Naphthalin oder Biphenyl, in inerten Lösungsmitteln, wie Ethern, Tetrahydrofuran (THF) oder Toluol, werden die 17β-Ether der Formel (XI) gebildet, worin R₁ ein C₁₋₆-Alkylrest oder ein CₙF₂ₙ₊₁-Rest ist, wobei n 1, 2 oder 3 darstellt und R₂ und Y die vorstehend gegebene Bedeutung haben. Die Verbindungen der allgemeinen Formel (XI) werden regioselektiv an der 5(10)-Doppelbindung epoxidiert. Die Epoxidation mit Wasserstoffperoxid und Hexachlor- oder Hexafluoraceton erfolgt vorzugsweise in Gegenwart von katalytischen Mengen eines tertiären Amins, wie Triethylamin oder Pyridin, wobei ein Gemisch aus 5α,10α-Epoxy- und 5β,10β-Epoxy-17α-Methyl-substituierten Estr-9(11)-en-3,3-dimethylketal der Formel (XII) 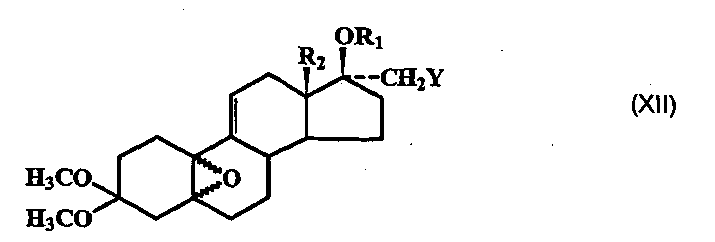 entsteht, worin R₁, R₂ und Y die vorstehend gegebene Bedeutung haben, das vorzugsweise nicht in die Einzelkomponenten getrennt wird, sondern direkt mit einem 4-Brombenzaldehydketal, wie 4-Brombenzaldehyddimethylketal, Magnesium und Cu(I)Cl bei Temperaturen zwischen -35°C und Raumtemperatur zu dem entsprechenden 3,3-Dimethoxy-5α-hydroxy-17α-(methyl-substituierten 11α,β-benzaldehyd-dimethylketal der Formel (XIII) 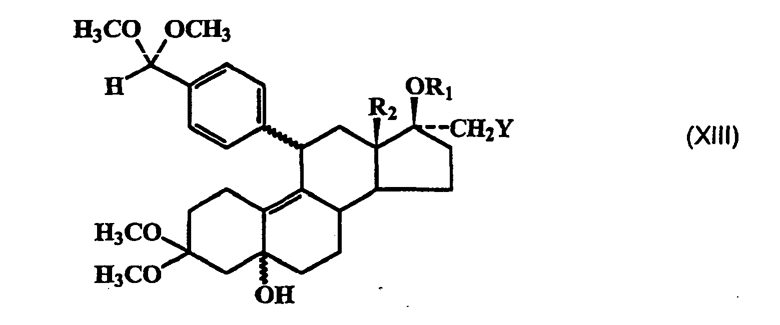 geöffnet wird, worin R₁, R₂ und Y die vorstehend gegebene Bedeutung haben. Dieses Gemisch wird bevorzugt direkt ohne Zwischenisolierung zur Abspaltung der Schutzgruppen der sauren Hydrolyse, beispielsweise mit verdünnter Essigsäure oder p-Toluolsulfonsäure, in Lösungsmitteln, wie Aceton oder THF, unterworfen. Dabei entsteht ein Gemisch der 11α,β-Benzaldehyd-Derivate der Formel (XIV) 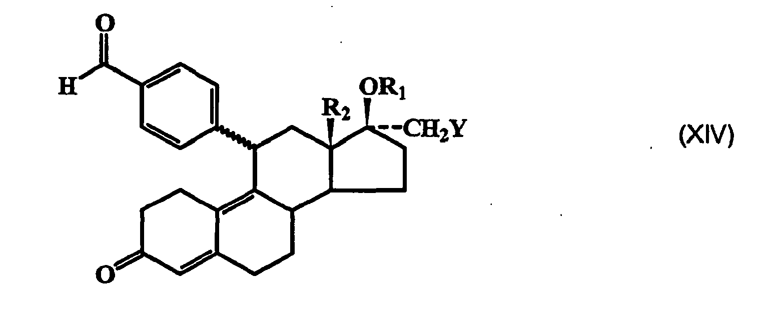 worin R₁, R₂ und Y die vorstehend gegebene Bedeutung haben, aus dem durch Kristallisation überraschenderweise (Das heißt, bei der Grignardierung des Epoxidgemisches enstehen am C-11 zwei isomere Verbindungen, die sich in ihren chromatographischen Eigenschaften nur gering unterscheiden und eigentlich auch sehr ähnlich kristallisieren sollten. Überraschenderweise kristallisiert nur die 11β-Verbindung aus, weil sie sehr schwer löslich ist. Somit gelingt durch eine einzige Kristallisation die weitgehende Abtrennung des 11-α-Aldehyds und gleichzeitig auch die Abtrennung der nichtsteroidalen Nebenprodukte. Dies ist besonders wichtig, da diese Nebenprodukte durch den vierfachen Überschuß an Grignardreagens ansonsten besonders störend wirken würden und nur durch Chromatographie abtrennbar wären) die reinen 11β-Benzaldehyde isoliert werden, worin R₁, R₂ und Y die vorstehend gegebene Bedeutung haben, und die Aldehydfunktion durch Hydroxylammoniumsalze, vorzugsweise Hydroxylaminhydrochlorid, in Gegenwart von Basen, vorzugsweise Pyridin, bei Raumtemperatur in ein Gemisch der E/Z-Benzaldoxime der allgemeinen Formel (I) umgewandelt werden, worin R₁, R₂ und Y die vorstehend gegebene Bedeutung haben und X eine E- oder Z-ständige OH-Gruppe bedeutet. Die E/Z-Benzaldoxime der allgemeinen Formel (I) können durch Umkristallisation und/oder durch Chromatographie getrennt, gereinigt und als Einzelkomponenten isoliert werden.

Unter "Alkylrest" wird in der vorliegenden Erfindung ein verzweigter oder geradkettiger Alkylrest verstanden. Als C₁₋₄- bzw C₁₋₆-Alkylreste seien beispielsweise eine Methyl-, Ethyl-, n-Propyl-, i-Propyl-, n-Butyl- , i-Butyl- oder tert.-Butyl-, n-Pentyl-, i-Pentyl-, n-Hexyl-, 2-Methylpentyl-, 3-Methylpentyl-, 2,2-Dimethylbutyl- oder 2,3-Dimethylbutylgruppe genannt. Unter CₙF₂ₙ₊₁-Rest wird ein verzweigter oder geradkettiger Fluoralkylrest mit 1 bis 3 Kohlenstoffatomen verstanden, wobei Beispiele eine Trifluormethyl-, Pentafluorethyl-, Heptafluor-n-propyl- oder Heptafluor-iso-propylgruppe sind. Bevorzugt bedeuten R₁ und R₂ einen C₁₋₃-Alkylrest, besonders bevorzugt eine Methylgruppe oder eine Trifluormethylgruppe.

Y bedeutet bevorzugt einen OC₁₋₃-Alkyl- oder SC₁₋₃-Alkylrest, besonders bevorzugt eine Methoxy-, Ethoxy-, Isopropyloxy-, Methylthio- oder Ethylthiogruppe, oder Trifluorethoxygruppe. Die Verbindungen der Formel (I), in denen R₁ ein CₙF₂ₙ₊₁-Rest ist und/oder Y eine OCH₂CₙF₂ₙ₊₁-Gruppe ist, sind neu.

Am stärksten bevorzugt sind im Rahmen von Verbindungen der Formel (I) die folgenden Verbindungen:
4-[17β-Hydroxy-17α-(methoxymethyl)-3-oxoestra-4,9-dien-11β-yl]benzaldehyd-1 E-oxim,
4-[17β-Hydroxy-17α-(methoxymethyl)-3-oxoestra-4,9-dien-11β-yl]benzaldehyd-1Z-oxim,
4-[17β-Methoxy-17α-(methoxymethyl)-3-oxoestra-4,9-dien-11β-yi]benzaidehyd-1E-oxim,
4-[17β-Methoxy-17α-(methoxymethyl)-3-oxoestra-4,9-dien-11β-yl]benzaldehyd-1Z-oxim,
4-[17β-Ethoxy-17α-(methoxymethyl)-3-oxoestra-4,9-dien-11β-yl]benzaldehyd-1E-oxim,
4-[17β-Hydroxy-17α-(ethoxymethyl)-3-oxoestra-4,9-dien-11β-yl]benzaldehyd-1E-oxim,
4-[17β-Methoxy-17α-(ethoxymethyl)-3-oxoestra-4,9-dien-11β-yl]benzaldehyd-1 E-oxim,
4-[17β-Hydroxy-17α-(isopropyloxymethyl)-3-oxoestra-4,9-dien-11β-yl]benzaldehyd-1 E-oxim,
4-[17β-Methoxy-17α-(isopropyloxy-methyl)-3-oxoestra-4,9-dien-11β-yl]benzaldehyd-1E-oxim,
4-[17β-Hydroxy-17α-(ethylthiomethyl)-3-oxoestra-4,9-dien-11β-yl]benzaldehyd-1 E-oxim und
4-[17β-Hydroxy-17α-(1,1,1-trifluorethoxymethyl)-3-oxoestra-4,9-dien-11 β-yl]benzaldehyd-1 E-oxim.

Die Verbindungen werden gut am Gestagenrezeptor gebunden, zeigen im Tierexperiment eine starke antigestagene Aktivität, besitzen eine partielle gestagene Aktivität und weisen nur eine geringe glucocorticoide Rezeptorbindung auf [siehe Tabelle 1 und vgl. DE 43 32 283 A1 (EP 0 648 778 B1)].

### Biologische Charakterisierung der erfindungsgemäßen Verbindungen

Die Rezeptorbindungsaffinität wurde durch kompetitive Bindung eines spezifisch bindenden ³H-markierten Tracers und der zu testenden Verbindung an Rezeptoren im Cytosol aus tierischen Target-Organen bestimmt. Dabei wurden Rezeptorsättigung und Reaktionsgleichgewicht bei folgenden Reaktionsbedingungen angestrebt:

### Progesteronrezeptor:

Uterus-Cytosol des Estradiol geprimten Kaninchens, aufbewahrt bei - 30 °C, in TED-Puffer (20 mM Tris/HCl, p_{H} 7,4; 1 mMol Ethylendiamintetraacetat, 2 mMol Dithiothreitol) mit 250 mMol Saccharose.
Tracer: ³H -ORG 2058
Refernzsubstanz : Progesteron

### Glucocorticoid-Rezeptor:

Thymus-Cytosol der adrenalektomierten Ratte, Thymi aufbewahrt bei -30°C; Puffer TED
Tracer: ³H-Dexamethason, 20 nM
Referenzsubstanz: Dexamethason

Die frühabortive Wirkung wurde bei der Ratte nach subcutaner Applikation vom 5. bis 7. Graviditätstag [Applikation 0,2 ml / Tier/ Tag in Benzylbenzoat / Rizinusöl (1 + 4 v/v)] ermittelt. Die Dosis gibt an, welche Menge mindestens je 4 Tieren im Vergleich zu unbehandelten Tieren gegeben werden muß, damit eine komplette Graviditätshemmung erreicht wird.

**Tabelle 1**

| **Rezeptorbindung von ausgewählten Verbindungen** | | | |
|---|---|---|---|
| Verbindung | Progester on-rezeptor [Progester -on =100 %] | Glucocorticoid-rezeptor [Dexamethason = 100 %] | 100 % frühabortive Wirkung bei der Ratte [mg/Tier/Tag] |
| 4-[17β-Hydroxy-(17α-(methoxymethyl)-3-oxoestra-4,9-dien-11β-yl]benzaldehyd-1 E-oxim | 165 | 76 | 1,0 |
| 4-[17β-Hydroxy-(17α-(methoxymethyl)-3-oxoestra-4,9-dien-11β-yl]benzalde-hyd-1 Z-oxim | 75 | 67 | 1,0 |
| 4-[17β-Methoxy-(17α-(methoxymethyl)-3-oxoestra-4,9-dien-11β-yl]benzaldehyd-1E-oxim | 302 | 78 | 1 ,0 |
| 4-[17β-Methoxy-17α-(methoxymethyl)-3-oxoestra-4,9-dien-11β-yl]benzaldehyde-1Z-oxime | 126 | 52 | 1,0 |
| 4-{17β-Hydroxy-17α-(1,1,1-trifluorethoxymethyl)-3-oxoestra-4,9-dien-11β-yl]benzaldehyd-1E-oxim | 177 | 94 | 3,0 |
| Ru 38 486 (Referenz) | 685 | 506 | 3,0 |

### Beispiel 1

### 4-[17β-Hydroxy-17α-(methoxymethyl)-3-oxoestra-4,9-dien-11β-yl]benzaldehyd-1E-oxim (la)

33 g 4-[17β-Hydroxy-17α-(methoxymethyl)-3-oxoestra-4,9-dien-11β-yl]benzaldehyd (VIIa) werden unter Argon in 250 ml Pyridin gelöst und mit 5,8 g Hydroxylsminhydrochlorid versetzt. Nach 2 Stunden wird in Eiswasser eingerührt, der Niederschlag wird abgesaugt, gewaschen und getrocknet. Das Rohprodukt (40 g) wird durch Chromatographie an Kieselgel gereinigt. Man erhält 20 g 4-[17β-Hydroxy-(17α-(methoxymethyl)-3-oxoestra-4,9-dien-11β-yl]benzaldehyd-1E-oxim (la) [Schmp. 135 bis 145°C (EtOH/Wasser); α_{D} = + 236° (CHCl₃); ¹H-NMR: 9,00 (s, 1H, NOH), 8,11 (s, 1H, HC=N), 7,45 (d, 2H, J = 8,2 , H-3'), 7,17 (d, 2H, J = 8,2 , H-2'), 5,79 (s, 1H, H-4), 4,38 (d, 1H, J = 7,1, H-11), 3,58 (d, 2H, J = 9,0, CH₂O), 3,43 (s, 3H, OCH₃), 3,25 (d, 2H, J = 9,0, CH₂O), 0,48 (s, 3H, H-18)] und 1,5 g 4-[17β-Hydroxy-17α-(methoxymethyl)-3-oxoestra-4,9-dien-11β-yl]benzaldehyd-1Z-oxim (Ib) [Schmp. 135 bis 146°C (Aceton); α_{D} = +192°; ¹H-NMR: 8,56 (s, 1H, NOH), 7,86 (d, 2H, J = 8,4 , H-3'), 7,33 (s, 1H, HC=N), 7,26 (d, 2H, J = 8,4 , H-2'), 5,79 (s, 1 H, H-4), 4,41 (d, 1 H, J = 7,2 , H-11), 3,57 (d, 2H, J = 9,1 , CH₂O), 3,42 (s, 3H, OCH₃), 3,23 (d, 2H, J = 9,1, CH₂O), 0,54 (s, 3H, H-18).

### Herstellung der Ausgangsverbindung

### Stufe 1

### 3,3-Dimethoxy-estra-5(10),9(11)-diene-17(S)-spiro-1',2'-oxirane (Xa)

12 g 3,3-Dimethoxy-estra-5(10), 9(11)-dien-17-on (IXa) und 9 g Trimethylsulfoniumjodid in 60 ml Dimethylformamid werden unter Rühren und leichter Kühlung mit 5,5 g Kalium-tert.-butylat versetzt. Nach 60 Minuten setzt man 50 ml n-Hexan und 30 ml Wasser zu, trennt die Phasen und wäscht die organische Phase mit Wasser. Die Lösung wird mit Natriumsulfat getrocknet und im Vakuum bis zur Kristallisation eingeengt. Die Umkristallisation erfolgt aus n-Hexan. Ausbeute: 11 g. Schmp. 94 bis 97°C; α_{D} = + 164° (CHCl₃); ¹H-NMR (δ, ppm, 300 MHz, CDCl₃/TMS): 5,51 (d, J = 5,6 Hz, H-11), 3,24 (s, 3H, OCH₃), 3,23 (s, 3H, OCH₃), 2,93 (d, 1H, J = 5,0 Hz, H-20), 2,66 (d, 1 H, J = 5,0 Hz, H-20), 0,87 (s, 2H, H-18).

### Stufe 2

### 3,3-Dimethoxy-17α-(methoxymethyl)-estra-5(10),9(11)-dien-17β-oi (XIa).

11 g der 3,3-Dimethoxy-estra-5(10),9(11)-diene-17(S)-spiro-1',2'-oxirane (Xa) werden in 40 ml Methanol suspendiert und mit 40 ml 3N Natriummethylatlösung versetzt und 2 Stunden am Rückfluß gekocht. Nach Zugabe von Wasser destilliert man das Methanol ab, extrahiert mit tert.-Butylmethylether und engt ein. Die Umkristallisation erfolgt aus einem Ethanol/Wasser-Gemisch. Schmp. 73 bis 76°C; α_{D} = +135° (CHCl₃); ¹H-NMR (δ, ppm), 5,56 (m, 1H, H-11), 3,47 (d, 1H, J = 9,1 Hz, OCH₂), 3,37 (s, 3H,CH₂OCH₃), 3,24 (s, 3H, 3-OCH₃), 3,23 (s, 3H, 3-OCH₃), 3,20 (d, 1 H, J = 9,1 Hz, OCH₂), 0,88 (s, 3H, H-18).

### Stufe 3

### 3,3-Dimethoxy-5α,10α-epoxy-17α-(methoxymethyl)-estr-9(11)-en-17β-ol und 3,3-Di-methoxy-5β,10β-epoxy-17α-(methoxymethyl)-estr-9(11)-en-17β-ol (XIIa):

Zu einer Lösung von 30 g (Xla) in 300 ml Methylenchlorid werden nacheinander 4,2 ml Pyridin, 3,75 ml Hexafluoraceton-Sesquihydrat und 37,5 ml 50 %-ige Wasserstoffperoxid-Lösung zugegeben. Nach 4 Stunden bei Raumtemperatur wird Natriumthiosulfat-Lösung zugegeben, die Phasen werden getrennt, die organische Phase mit Natriumbicarbonat-Lösung und Wasser gewaschen, getrocknet und das Lösungsmittel im Vakuum verdampft. Man erhält 31 g Rohprodukt (XIIa), das direkt bei der Grignardreaktion eingesetzt wird.

### Stufe 4

### 4-[(3,3-Dimethoxy)-5α,17β-dihydroxy-17α-(methoxymethyl)-estr-9-en-11α,β-yl]benzaldehyd-dimethylketal (XIIIa):

Zu einer Grignard-Lösung, die aus 7,6 g Magnesium, 72 g 4-Brombenzaldehyd-dimethylketal in 100 ml THF hergestellt wird, werden bei -35 °C 4,5 g Kupfer-I-chlorid zugegeben. Man rührt 20 Minuten bei dieser Temperatur und tropft dann eine Lösung von 28 g Epoxidgemisch (XIIa) in 70 ml THF zu. Anschließend läßt man auf Raumtemperatur erwärmen, versetzt mit wäßriger Ammoniumchlorid-Lösung und extrahiert das Steroid mit Essigester, wäscht die organische Phase neutral, trocknet sie und engt im Vakuum ein. Das Rohprodukt (XIIIa) wird direkt in der nächsten Stufe eingesetzt.

### Stufe 5

### 4-[17β-Hydroxy-17α-(methoxymethyl)-3-oxoestra-4,9-dien-11α,β-yl]benzaldehyd (IVXa):

75 g Rohprodukt (XIIIa) werden in 250 ml 70 %-lger Essigsäure gelöst und unter Argon 2 Stunden bei 50 °C gerührt. Man kühlt ab, setzt Methylenchlorid zu, trennt die Phasen, wäscht neutral, fügt Methyl-tert.-butylether zu und engt die organische Phase im Vakuum ein. Man erhält 24 g eines blaßgelben Rohprodukts (XIVa), das nur noch einen kleinen Teil des 11α-Benzaldehyds enthält.

### Stufe 6

### 4-[17β-Hydroxy-17α-(methoxymethyl)-3-oxoestra-4,9-dien-11β-yl]benzaldehyd (VIIa):

24 g Rohprodukt (XIVa) werden in Methylenchlorid gelöst, mit tert.-Butylmethylether versetzt und im Vakuum eingeengt. Dabei fällt der reine 11β-Benzaldehyd (VIIa) kristallin aus, der erneut in Essigester umkristallisiert wird.

Schmp. 235 bis 240° C; α_{D} = +209° (CHCl₃); ¹H-NMR: 9,97 (s, 1H, CHO), 7,80 (d, 2H, J = 8,1, H-3'), 7,38 (d, 2H, J = 8,1, H-2'), 5,80 (s, 1H, H-4), 4,45 (d, 1H, J = 7,5, H-11), 3,57 (d, 2H, J ,= 9,2, CH₂O), 3,42 (d, 2H, J = 10,8, CH₂O), 3,41 (s, 3H, OCH₃), 0,51 (s, 3H, H-18).

### Beispiel 2

### 4-[17β-Methoxy-17α-(methoxymethyl)-3-oxoestra-4,9-dien-11β-yl]benzaldehyd-1E-oxim (Ic)

Zu einer Lösung von 10 g (VIIb) in 100 ml Pyridin werden bei Raumtemperatur 1,75 g Hydroxylaminhydrochlorid zugegeben und die Mischung wird 2 Stunden gerührt. Man gießt in Eiswasser ein, saugt den Niederschlag ab, trocknet über Calciumchlorid und chromatographiert das Rohprodukt an Kieselgel. Man isoliert 7g 4-[17β-Methoxy-17α-(methoxymethyl)-3-oxoestra-4,9-dien-11β-yl]benzaldehyd-1E-oxim (Ic). [Schmp. 196-198°C (EtOH/H₂O); α_{D} = + 220° (CHCl₃); ¹H-NMR: 8,38 (s, 1H, NOH), 8,10 (s, 1H, HC=N), 7,47 (d, 2H, J = 8,1, H-3'), 7,20 (d, 2H, J = 8,1, H-2'), 5,79 (s, 1H, H-4), 4,38 (d, 1H, J = 7,3, H-11), 3,58 (d, 2H, J = 10,8, CH₂O), 3,41 (s, 3H, OCH₃), 3,41 (d, 2H, J = 10,8 , CH₂O), 3,25 (s, 3H, OCH₃), 0,54 (s, 3H, H-18)] und 300 mg 4-[17β-Methoxy-17α-(methoxymethyl)-3-oxoestra-4,9-dien-11β-yl]benzaldehyd-1Z-oxim (Id) [Schmp. 120-138 °C (Aceton/n-Hexan); α_{D} = +217° (CHCl₃); ¹H-NMR: 9,38 (s, 1H, NOH), 7,88 (d, 2H, J = 8,9, H-3'), 7,33 (s, 1H, HC=N), 7,26 (d, 2H, J = 8,9, H-2'), 5,79 (s, 1H, H-4), 4,39 (d, 1H, J = 7,3, H-11), 3,58 (d, 2H, J = 10,5, CH₂O), 3,42 (d, 2H, J = 10,5, CH₂O), 3,41 (s, 3H, OCH₃), 3,26 (s, 3H, OCH₃), 0,54 (s, 3H, H-18).

### Herstellung der Ausgangsverbindung

### Stufe 1

### 3,3,17β-Trimethoxy-17α-(methoxymethyl)-estra-5(10,9(11)-dien (XIb)

12 g (Xla), 5 ml Methyljodid und 10 g Kalium-tert.-butylat werden in 80 ml tert.-Butylmethylether 4 Stunden bei 30°C bis 40°C gerührt. Nach Zugabe von Wasser erfolgt die Phasentrennung, die organische Phase wird mit Wasser gewaschen, getrocknet und eingeengt. Das Rohprodukt (Xlb) wird in Methanol umkristallisiert.

Schmp. 95 bis 97°C (MeOH); α_{D} = +146° (CHCl₃); ¹H-NMR (δ, ppm): 5,52 (m, 1H, H-11), 3,63 (d, 1H, J = 10,7 Hz, OCH₂), 3,38 (s, 3H, CH₂OCH₃), 3,31 (d, 1H, J = 10,7 Hz, OCH₂), 3,29 (s, 3H, 17β-OCH₃), 3,24 (s, 3H, 3-OCH₃), 3,23 (s, 3H, 3-OCH₃), 0,88 (s, 3H, H-18).

### Stufe 2

### 17α-(Methoxymethyl)-3,3,17β-trimethoxy-5α,10α-epoxy-estr-9(11)-en und 17α-(Methoxymethyl)-3,3,17β-trimethoxy-5β,10β-epoxy-estr-9(11)-en (XIIb):

Zu 2g (XIb) in 20 ml Methylenchlorid werden 0,3 ml Pyridin und 0,25 ml Hexaflucraceton-Sesquihydrat zugegeben, Bei Raumtemperatur werden 2,5 ml Wasserstoffperoxid zugetropft, nach 4 Stunden wird Natriumsulfit-Lösung zugegeben, die Phasen werden getrennt und die organische Phase mit Natriumbicarbonat und Wasser gewaschen, über Natriumsulfat getrocknet und unter Vakuum verdampft. Das Epoxidgemisch (XIIb) wird direkt in der nächsten Stufe eingesetzt.

### Stufe 3

### 11α,β-4-[17α-(Methoxymethyl)-3,3,17β-trimethoxy-5α-hydroxy-estr-9-en-11β-yl]benzaldehyd-dimethylketal (XIIIb)

Zu einer Grignard-Lösung, die aus 2,4 g 4-Brombenzaldehyddimethylketal, 0,2 g Magnesium in 20 ml THF hergestellt wird, werden bei -35°C 60 mg Kupfer(I)chlorid zugegeben. Man rührt 20 Minuten bei dieser Temperatur und tropft eine Lösung von 1 g (XIIb) in 5 ml THF zu. Anschließend läßt man die Reaktion auf Raumtemperatur kommen, zersetzt den Ansatz mit wäßriger Ammoniumchlorid-Lösung, extrahiert die Lösung mit Essigester und wäscht die organische Phase mit Wasser, trocknet sie mit Natriumsulfat und engt im Vakuum ein. Das Rohprodukt (XIIIb) (1,5 g) wird direkt in der nächsten Stufe eingesetzt.

### Stufe 4

### 4-[17β-Methoxy-17α-(methoxymethyl)-3-oxoestra-4,9-dien-11β-yl]benzaldehyd (VIIb):

1,5 g Rohprodukt (XIIIb) werden in 20 ml Aceton gelöst und mit 180 mg p-Toluolsulfonsäure versetzt. Nach 1 Stunde wird mit wäßrigem Ammoniak neutralisiert und mit Wasser verdünnt. Dabei fällt das Aldehydgemisch (XIVB) aus, das aus Aceton umkristallisiert wird. Man erhält 0,7 g 4-[17β-Methoxy-17α-methoxymethyl)-3-oxoestra-4,9-dien-11β-yl]benzaldehyd (VIIb). Schmp. 245 bis 250°C (Aceton); α_{D} = +193° (CHCl₃); ¹H-NMR: 9,97 (s, 1H, CHO), 7,79 (d, 2H, J = 8,1, H-3'), 7,37 (d, 2H, J = 8,1, H-2'), 5,79 (s, 1H, H-4), 4,44 (d, 1H, J = 7,5, H-11), 3,56 (d, 2H, J = 10,8, CH₂O), 3,42 (d, 2H, J = 10,8, CH₂O), 3,41 (s, 3H, OCH₃), 3,25 (s, 3H, OCH₃), 0,51 (s, 3H, H-18),

### Beispiel 3

### 4-[17β-Ethoxy-17α-(methoxymethyl)-3-oxoestra-4,9-dien-11β-yl]benzaldehyd-1E-oxim (Ie)

1,7 g (VIIc) werden in 25 ml Pyridin mit 250 mg Hydroxylaminhydrochlorid 1 Stunde bei Raumtemperatur gerührt. Danach gießt man in 100 ml Eiswasser ein, saugt den Niederschlag ab, wäscht mit Wasser neutral und trocknet mit Calciumchlorid. Das Rohprodukt (1,7g) wird durch Chromatographie an Kieselgel gereinigt. Man erhält 890 mg (le). Schmp. 184 bis 187 °C (Aceton/Hexan); α_{D} = +214° (CHCl₃); ¹H-NMR: 9,10 (s, 1H, CH=N), 7,58 (s,1H, OH), 7,49 (d, 2H, J = 8,4, H-3'), 7,21 (d, 2H, J = 8,4, H-2'), 5,78 (s, 1 H, H-4), 4,38 (d, 1H, J = 6,9, H-11), 3,62 (d, 2H, J = 10,8, CH₂O), 3,40 (s, 3H, OCH₃), 3,36 (d, 2H, J = 10,8, CH₂O), 1,11 (t, 3H, CH₂CH₃), 0,54 (s, 3H, H-18).

### Herstellung der Ausgangsverbindung

### Stufe 1

### 3,3,-Dimethoxy-17α-(methoxymethyl)-estra-5(10),9(11)-dien-17β-ethoxymethylether (XIc)

4,2 g (XIa) werden mit 15,6 g Kalium-tert.-butanolat und 76 ml lodethan in 400 ml Toluol bei 35°C innerhalb von 14 Stunden umgesetzt. Nach Zugabe von Wasser werden die Phasen getrennt, die organische Phase neutral aufgearbeitet und das Lösungsmittel nach Trocknung im Vakuum verdampft. Das Rohprodukt (XIc) wird direkt ohne Reinigung in der nächsten Stufe eingesetzt.

### Stufe 2

### 3,3,-Dimethoxy-5α,10α-epoxy- 17α-(methoxymethyl)-estra-5(10),9(11)-dien-17β-ethoxymethylether und 3,3,-Dimethoxy-5β,10β-epoxy-17α-(methoxymethyl)-estra-5(10),9(11)-dien-17β-ethoxymethylether (XIIc)

Zu 3,3 g (Xlc) in 20 mi Methylenchlorid werden 0,5 mi Pyridin und ü,4 ml Hexafluoraceton-Sesquihydrat zugegeben. Bei Raumtemperatur werden 4,5 ml Wasserstoffperoxid zugetropft, nach 4 Stunden wird Natriumsulfit-Lösung zugegeben, die Phasen werden getrennt und die organische Phase mit Natriumbicarbonat und Wasser gewaschen, über Natriumsulfat getrocknet, und unter Vakuum verdampft. Das **Epoxidgemisch (XIIc) wird direkt in der nächsten Stufe eingesetzt.**

### Stufe 3

### 4-[3,3-Dimethoxy-17β-ethoxy-5α-hydroxy-17α-(methoxymethyl)-estr-9-en-11α,β-yl]benZaldehyd-1,1-dimethylketal (XIIIc)

Zu einer Grignard-Lösung, die aus 4,0 g 4-Brombenzaldehyddimethylketal, 0,3 g Magnesium in 20 ml THF hergestellt wird, werden bei -35 °C 60 mg Kupfer(I)chlorid zugegeben. Man rührt 20 Minuten bei dieser Temperatur und tropft eine Lösung von 2 g (XIIc) in 5 ml THF zu. Anschließend läßt man die Reaktion auf Raumtemperatur kommen, zersetzt den Ansatz mit wäßriger Ammoniumchlorid-Lösung, extrahiert die Lösung mit Essigester und wäscht die organische Phase mit Wasser, trocknet sie mit Natriumsulfat und engt im Vakuum ein. Das Rohprodukt (XIIIc) (4,5 g) wird direkt in der nächsten Stufe eingesetzt.

### Stufe 4

### 4-[17β-Ethoxy-17α-(methoxymethyl)-3-oxoestra-4,9-dien-11β-yl]benzaldehyd (VIIc):

2,8 g (Xlllc) werden in 40 ml Aceton gelöst. Nach Zugabe von 1,0 ml Wasser werden 1,4 g p-Toluolsulfonsäure zugesetzt und nach 1 Stunde wird Eiswasser zugegeben. Der dabei ausfallende (VIIc) wird abgesaugt, getrocknet und in Aceton/Hexan und erneut in tert.-Butylmethylether umkristallisiert.

Schmp. 164 bis 167 °C; α_{D} = +199° (CHCl₃); ¹H-NMR: 9,97 (s, 1H, CHO), 7,80 (d, 2H, J = 8,1, H-3'), 7,37 (d, 2H, J = 8,1, H-2'), 5,79 (s, 1H, H-4), 4,43 (d, 1H, J = 7,5, H-11), 3,58 (d, 2H, J = 10,8, CH₂O), 3,41 (m, 2H, CH₂O), 3,40 (s, 3H, OCH₃), 1,10 (t, 3H, Ethyl), 0,51 (s, 3H, H-18).

### Beispiel 4

### 4-[17β-Methoxy-17α-(ethoxymethyl)-3-oxoestra-4,9-dien-11β-yl]benzaldehyd-1E-oxim

Die Herstellung erfolgt analog Beispiel 2. Schmp. 90-95 °C (tert.-Butylmethylether) α_{D} = + 177° (CHCl₃); ¹H-NMR: 8,10 (s, 1H, HC=N), 7,60 (s, 1H, NOH), 7,48 (d, 2H, J = 8,1, H-3'), 7,20 (d, 2H, J = 8,1, H-2'), 5,78 (s, 1H, H-4), 4,36 (d, 1H, J = 7,3, H-11), 3,61 (d, 2H, J = 10,8, CH₂O), 3,42 (d, 2H, J = 10,8, CH₂O), 3,26 (s, 3H, OCH₃), 1,27 (t, 3H, Ethyl), 0,53 (s, 3H, H-18)

### Beispiel 5

### 4-[17β-Hydroxy-17α-(ethoxymethyl)-3-oxoestra-4,9-dien-11β-yl]benzaldehyd-1E-oxim

Die Herstellung erfolgt analog Beispiel 1.

Schaum (Hexan); α_{D} = + 226° (CHCl₃); ¹H-NMR: 8,10 (s, 1H, HC=N), 7,70 (s, 1H, NOH), 7,48 (d, 2H, J = 8,1, H-3'), 7,20 (d, 2H, J = 8,1, H-2'), 5,78 (s, 1H, H-4), 4,38 (d, 1H, J = 7,2, H-11), 3,61 (d, 2H, J = 10,8, CH₂O), 3,23 (d, 2H, J = 10,8 , CH₂O), 3,26 (s, 3H, OCH₃), 1,25 (t, 3H, Ethyl), 0,52 (s, 3H, H-18)

### Beispiel 6

### 4-[17β-Methoxy-(17α-(isopropyloxymethyl)-3-oxoestra-4,9-dien-11β-yl]benz-aldehyd-1E-oxim

Die Herstellung erfolgt analog Beispiel 1.

Schmp. 192-196 °C Zers. (Diethylether); α_{D} = + 215° (CHCl₃); ¹H-NMR: 8,10 (s, 1H, HC=N), 8,07 (s, 1H, NOH), 7,47 (d, 2H, J = 8,1, H-3'), 7,19 (d, 2H, J = 8,1, H-2'), 5,79 (s, 1H, H-4), 4,38 (d, 1H, J = 6,6, H-11), 3,62 (d, 2H, J = 10,8, CH₂O), 3,22 (d, 2H, J = 10,8, CH₂O), 3,02 (s, 1H, OH), 1,22 (m, 6H, Isopropyl), 0,52 (s, 3H, H-18)

### Beispiel 7

### 4-[17β-Hydroxy-(17α-(isopropyloxymethyl)-3-oxoestra-4,9-dien-11 β-yl]benz-aldehyd-1E-oxim

Die Herstellung erfolgt analog Beispiel 2. Schmp. 143 °C Zers. (Aceton/n-Hexan); α_{D} = + 199° (CHCl₃); ¹H-NMR: 8,10 (s, 1H, HC=N), 8,00 (s, 1H, NOH), 7,48 (d, 2H, J = 8,4, H-3'), 7,21 (d, 2H, J = 8,4, H-2'), 5,79 (s, 1H, H-4), 4,37 (d, 1H, J = 6,9, H-11), 3,61 (d, 2H, J = 10,5, CH₂O), 3,43 (d, 2H, J = 10,8, CH₂O), 3,26 (s, 3H, OCH₃), 1,22 (t, 6H, 6,0 Isopropyl), 0,54 (s, 3H, H-18)

### Beispiel 8

### 4-[17β-Hydroxy-(17α-(ethylthiomethyl)-3-oxoestra-4,9-dien-11β-yl]benzaldehyd-1E-oxim

Die Herstellung erfolgt analog Beispiel 1.

Schmp. 132 bis 137°C (Aceton); α_{D} = + 165° (CHCl₃); ¹H-NMR: 8,10 (s, 1H, HC=N), 7,93 (s, 1 H, NOH), 7,49 (d, 2H, J = 8,4, H-3'), 7,20 (d, 2H, J = 8,4, H-2'), 5,79 (s, 1H, H-4), 4,42 (d, 1H, J = 7,2, H-11), 2,96 (d, 2H, J = 13,2, CH₂S), 2,90 (s,1 H,OH), 2,70 (d, 2H, J = 12,9 , CH₂ S), 1,29 (t, 3H, 10,2, SCH₂CH₃), 0,56 (s, 3H, H-18)

### Beispiel 9

### 4-[17β-Hydroxy-(17α-(1,1,1-trifluorethoxymethyl)-3-oxoestra-4,9-dien-11β-yl]benz-aidehyd-1E-oxim

Die Herstellung erfolgt analog Beispiel 1.

Schmp. 132 bis 136°C (Diethylether), α_{D} = + 182° (CHCl₃); ¹H-NMR: 8,11 (s, 1H, HC=N), 7,60 (s, 1H, NOH), 7,49 (d, 2H, J = 8,4, H-3'), 7,20 (d, 2H, J = 8,1, H-2'), 5,79 (s, 1H, H-4), 4,41 (d, 1H, J = 7,2, H-11), 3,93 (m, 2H, CH₂CH₃), 3,82 (d, 2H, J = 9,0, CH₂O), 3,50 (m, CH₂CF₃+OH), 0,55 (s, 3H, H-18)

## Patentansprüche

1. Verfahren zur Herstellung von 4-(17α-Methyl-substituierten 3-Oxoestra-4,9-dien-11β-yl)benzaldehyd-(1E oder 1Z)-oximen der allgemeinen Formel (I) 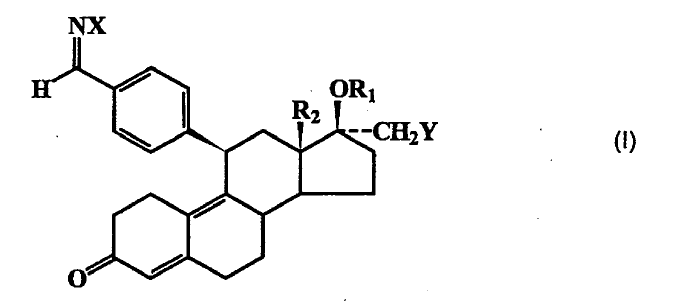 worin R₁ ein Wasserstoffatom, ein C₁₋₆-Alkylrest oder ein CₙF₂ₙ₊₁-Rest ist, wobei n 1, 2 oder 3 ist, R₂ ein C₁₋₄-Alkylrest oder eine Trifluormethylgruppe ist, X eine E- oder Z-ständige OH-Gruppe ist und Y eine OC₁₋₆-Alkylgruppe, SC₁₋₆-Alkylgruppe oder OCH₂CₙF₂ₙ₊₁-Gruppe ist, wobei n 1, 2 oder 3 ist, **dadurch gekennzeichnet, daß** das 3,3-Dimethoxy-estra-5(10),9(11)-dien-17-on der Formel (IX) 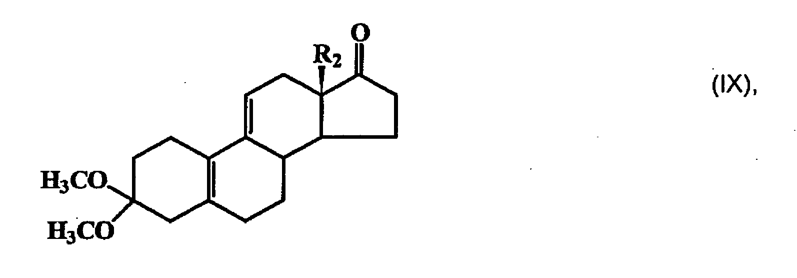 in der R₂ die vorstehend gegebene Bedeutung hat, mit einem aktiven Methylenreagenz in einem inerten Lösungsmittel in das Spiroepoxid der Formel (X) 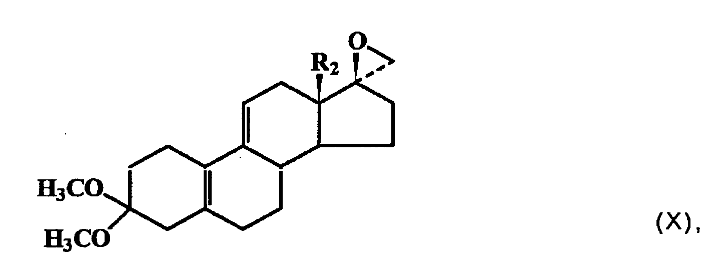 in der R₂ die vorstehend gegebene Bedeutung hat, umgewandelt wird und das nach Spaltung der 17-Spiroepoxygruppe durch Alkali- oder Erdalkalialkoholat, Alkali- oder Erdalkalithiolalkoholat oder durch Trifluoralkylalkohole und Kalium-tert.-butanolat in einem inerten Lösungsmittel zur 17α-CH₂Y-Verbindung der Formel (XI) 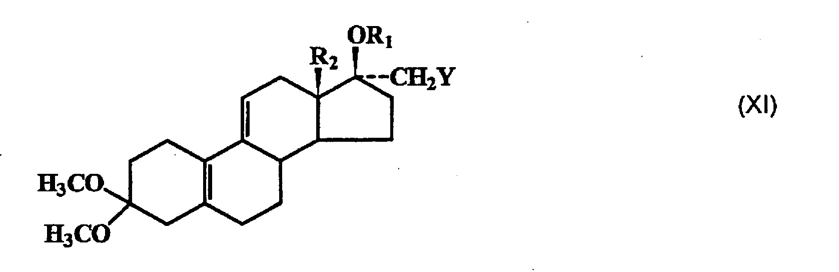 geöffnet wird, in der R₁ ein Wasserstoffatom darstellt und R₂ und Y die vorstehend gegebene Bedeutung haben, und diese Verbindung gegebenenfalls durch Umsetzung der 17β-Hydroxylgruppe mit Alkylhalogeniden oder Fluoralkyliodid in Gegenwart von starken Basen in einem inerten Lösungsmittel zu den 17β-Ethern der Formel (XI) umgesetzt wird, worin R₁ einen C₁₋₆-Alkylrest oder einen CₙF₂ₙ₊₁-Rest darstellt und R₂ und Y die vorstehend gegebene Bedeutung haben, die Verbindung der Formel (XI) an der 5(10)-Doppelbindung epoxidiert wird, wobei ein Gemisch aus 5α,10α-Epoxy- und 5β,10β-Epoxy-17α-methyl-substituierten Estr-9(11)-en-3,3-dimethylketal der Formel (XII) 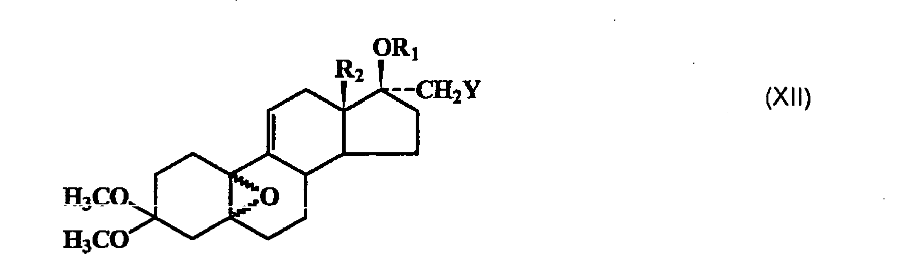 entsteht, worin R₁, R₂ und Y die vorstehend gegebene Bedeutung haben, das mit einem 4-Brombenzaldehydketal, Magnesium und Cu(I)Cl bei Temperaturen zwischen -35°C und Raumtemperatur zu dem entsprechenden 3,3-Dimethoxy-5α-hydroxy-17α-methyl-substituierten 11α,β-Benzaldehyddimethyl-ketal der Formel (XIII) 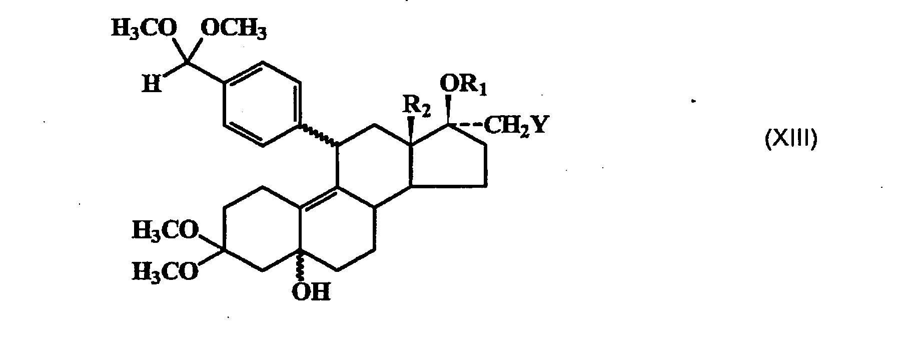 geöffnet wird, in dem R₁, R₂ und Y die vorstehend gegebene Bedeutung haben, und das zur Abspaltung der Schutzgruppen der sauren Hydrolyse unterworfen wird, wobei ein Gemisch der 11α,β-Benzaldehyd-Derivate der Formel (XIV) 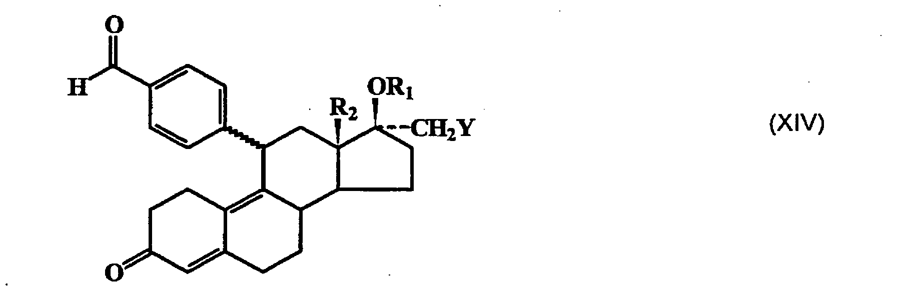 entsteht, worin R₁, R₂ und Y die vorstehend gegebene Bedeutung haben, aus dem durch Kristallisation die reinen 11β-Benzaldehyde der Formel (VII) isoliert werden, worin R₁, R₂ und Y die vorstehend gegebene Bedeutung haben, und die Aldehydfunktion durch Hydroxylammoniumsalze in Gegenwart von Basen, vorzugsweise Pyridin, bei Raumtemperatur in ein Gemisch der E/Z-Benzaldoxime der allgemeinen Formel (I) umgewandelt wird.

2. Verfahren nach Anspruch 1, **dadurch gekennzeichnet, daß** das Gemisch der E/Z-Benzaldoxime der allgemeinen Formel (I) durch Umkristallisation und/oder durch Chromatographie, in die Einzelkomponenten getrennt wird.

3. Verfahren nach einem der Ansprüche 1 oder 2, **dadurch gekennzeichnet, daß** R₁ einen C₁₋₃-Alkylrest, vorzugsweise eine Methylgruppe, oder Trifluormethylgruppe bedeutet.

4. Verfahren nach einem der Ansprüche 1 bis 3, **dadurch gekennzeichnet, daß** R₂ einen C₁₋₃-Alkylrest, vorzugsweise eine Methylgruppe bedeutet.

5. Verfahren nach einem der Ansprüche 1 bis 4, **dadurch gekennzeichnet, daß** Y einen OC₁₋₃-Alkyl- oder SC₁₋₃-Alkylrest, vorzugsweise eine Methoxy-, Ethoxy-, Isopropyloxy-, Methylthio- oder Ethylthiogruppe, oder 1, 1, 1- Trifluorethoxygruppe bedeutet.

6. Verfahren nach einem der Ansprüche 3 bis 5, **dadurch gekennzeichnet, daß** die folgenden Verbindungen hergestellt werden:
4-[17β-Hydroxy-17α-(methoxymethyl)-3-oxoestra-4,9-dien-11β-yl]benzaldehyd-1E-oxim,
4-[17β-Hydroxy-17α-(methoxymethyl)-3-oxoestra-4,9-dien-11β-yl]benzaldehyd-1Z-oxim.
4-[17β-Methoxy-17α-(methoxymethyl)-3-oxoestra-4,9-dien-11β-yl]benzaldehyd-1 E-oxim,
4-[17β-Methoxy-17α-(methoxymethyl)-3-oxoestra-4,9-dien-11β-yl]benz-aldehyd-1Z-oxim,
4-[17β-Ethoxy-(17α-(methoxymethyl)-3-oxoestra-4,9-dien-11β-yl]benzaldehyd-1E-oxim,
4-[17β-Hydroxy-17α-(ethoxymethyl)-3-oxoestra-4,9-dien-11β-yl]benzaldehyd-1 E-oxim,
4-[17β-Methoxy-17α-(ethoxymethyl)-3-oxoestra-4,9-dien-11β-yl]benzaldehyd-1 E-oxim,
4-[17β-Hydroxy-17α-(isopropyloxymethyl)-3-oxoestra-4,9-dien-11 β-yl]benzaldehyd-1E-oxim,
4-[17β-Methoxy-17α-(isopropyloxymethyl)-3-oxoestra-4,9-dien-11 β-yl]benzaldehyd-1E-oxim,
4-[17β-Hydroxy-17α-(ethylthiomethyl)-3-oxoestra-4,9-dien-11β-yl]benzaldehyd-1 E-oxim und
4-{17β-Hydroxy-17α-(1,1,1-trifluorethoxymethyl)-3-oxoestra-4,9-dien-11β-yl]benzaldehyd-1E-oxim.

7. Verbindungen der Formel (I), **dadurch gekennzeichnet, daß** R₁. ein CₙF₂ₙ₊₁-Rest ist, wobei n 1, 2 oder 3 ist, R₂, X und Y die in Anspruch 1 gegebene Bedeutung haben.

8. Verbindungen der Formel (I), **dadurch gekennzeichnet, daß** Y eine OCH₂CₙF₂ₙ₊₁-Gruppe ist, wobei n 1, 2 oder 3 ist, R₁, R₂ und X die in Anspruch 1 gegebene Bedeutung haben.

## Claims

1. Process for the production of 4-(17α-methyl-substituted 3-oxoestra-4,9-dien-11β-yl)benzaldehyde-(1E or 1Z)-oximes of general formula (I) 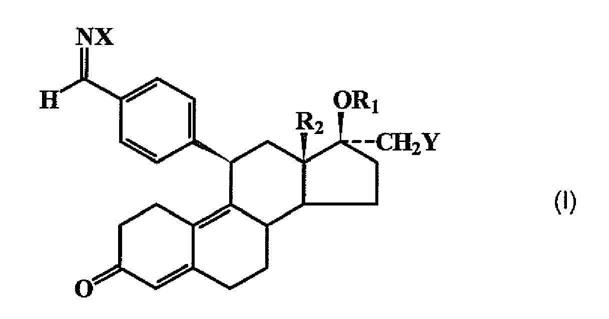 in which R₁ is a hydrogen atom, a C₁₋₆-alkyl radical or a CₙF₂ₙ₊₁ radical, whereby n is 1, 2 or 3, R₂ is a C₁₋₄-alkyl radical or a trifluoromethyl group, X means an OH group in E- or Z-position, and Y is an OC₁₋₆-alkyl group, an SC₁₋₆-alkyl group or an OCH₂CₙF₂ₙ₊₁ group, whereby n is 1, 2 or 3, **characterized in that** the 3,3-dimethoxy-estra-5(10),9(11)-dien-17-one of formula (IX) 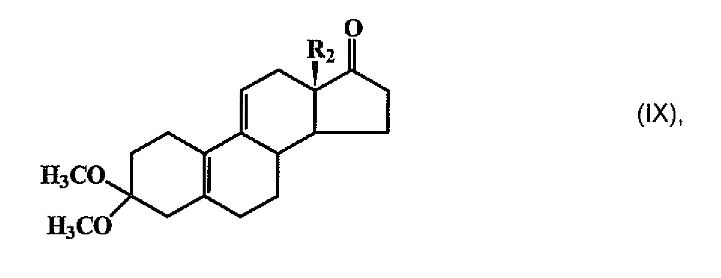 in which R₂ has the above-indicated meaning, is converted with an active methylene reagent in an inert solvent into the spiroepoxide of formula (X) 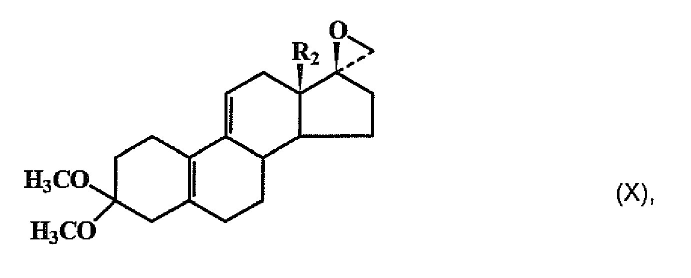 in which R₂ has the above-indicated meaning, and which, after cleavage of the 17-spiroepoxy group by alkali or alkaline-earth alcoholate, alkali or alkaline-earth thiol alcoholate or by trifluoroalkyl alcohols and potassium-tert-butanolate in an inert solvent, is opened to the 17α-CH₂Y compound of formula (XI) 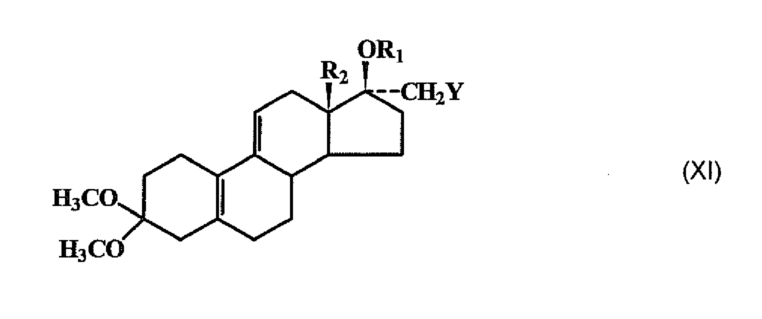 in which R₁ represents a hydrogen atom and R₂ and Y have the above-indicated meanings, and this compound optionally is reacted by reaction of the 17β-hydroxyl group with alkyl halides or fluoroalkyl iodide in the presence of strong bases in an inert solvent to form the 17β-ethers of formula (XI), in which R₁ represents a C₁₋₆-alkyl radical or a CₙF₂ₙ₊₁ radical, and R₂ and Y have the above-indicated meanings, the compound of formula (XI) is epoxidized on the 5(10)-double bond, whereby a mixture is produced from 5α,10α-epoxy- and 5β,10β-epoxy-17α-methyl-substituted estr-9(11)-ene-3,3-dimethtyl ketal of formula (XII) 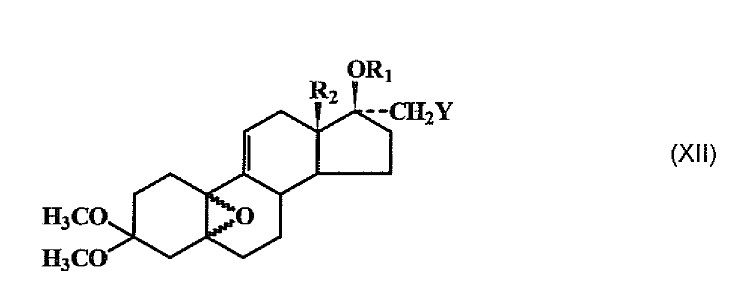 in which R₁, R₂ and Y have the above-indicated meanings, which is opened with a 4-bromobenzaldehyde ketal, magnesium and Cu(I)Cl at temperatures of between -35°C and room temperature to the corresponding 3,3-dimethoxy-5α-hydroxy-17α-methyl-substituted 11 α,β-benzaldehydedimethyl ketal of formula (XIII) 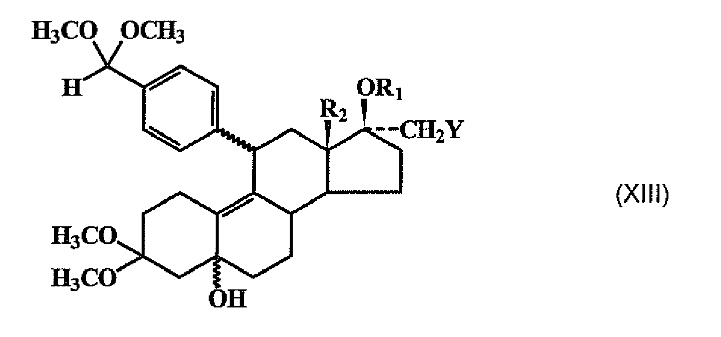 in which R₁, R₂ and Y have the above-indicated meanings, and which is subjected to acid hydrolysis to cleave the protective groups, whereby a mixture of the 11α,β-benzaldehyde derivatives of formula (XIV) 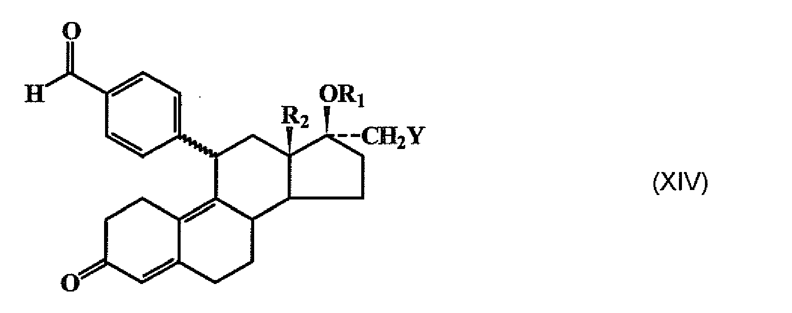 is produced, in which R₁, R₂ and Y have the above-indicated meanings, from which the pure 11β-benzaldehydes of formula (VII) are isolated by crystallization, in which R₁, R₂ and Y have the above-indicated meanings, and the aldehyde function is converted by hydroxylammonium salts in the presence of bases, preferably pyridine, at room temperature into a mixture of the E/Z-benzaldoximes of general formula (I).

2. Process according to claim 1, wherein the mixture of the E/Z-benzaldoximes of general formula (I) is separated into the individual components by recrystallization and/or by chromatography.

3. Process according to one of claims 1 or 2, wherein R₁ means a C₁₋₃-alkyl radical, preferably a methyl group or a trifluoromethyl group.

4. Process according to one of claims 1 to 3, wherein R₂ means a C₁₋₃-alkyl radical, preferably a methyl group.

5. Process according to one of claims 1 to 4, wherein Y means an OC₁₋₃-alkyl radical or an SC₁₋₃-alkyl radical, preferably a methoxy, ethoxy, isopropyloxy, methylthio or ethylthio group, or a trifluoroethoxy group.

6. Process according to one of claims 3 to 5, wherein the following compounds are produced:
4-[17β-Hydroxy-17α-(methoxymethyl)-3-oxoestra-4,9-dien-11β-yl]benzaldehyde-1E-oxime,
4-[17β-Hydroxy-17α-(methoxymethyl)-3-oxoestra-4,9-dien-11β-yl]benzaldehyde-1Z-oxime,
4-[17β-Methoxy-17α-(methoxymethyl)-3-oxoestra-4,9-dien-11β-yl]benzaidehyde-1E-oxime,
4-[17β-Methoxy-17α-(methoxymethyl)-3-oxoestra-4,9-dien-11β-yl]benzaldehyde-1Z-oxime,
4-[17β-Ethoxy-(17α-(methoxymethyl)-3-oxoestra-4,9-dien-11β-yl]benzaldehyde-1E-oxime,
4-[17β-Hydroxy-17α-(ethoxymethyl)-3-oxoestra-4,9-dien-11β-yl]benzaldehyde-1E-oxime,
4-[17β-Methoxy-17α-(ethoxymethyl)-3-oxoestra-4,9-dien-11β-yl]benzaldehyde-1E-oxime,
4-[17β-Hydroxy-17α-(isopropyloxymethyl)-3-oxoestra-4,9-dien-11β-yl]benzaldehyde-1E-oxime,
4-[17β-Methoxy-17α-(isopropyloxymethyl)-3-oxoestra-4,9-dien-11β-yl]benzaldehyde-1E-oxime,
4-[17β-Hydroxy-17α-(ethylthiomethyl)-3-oxoestra-4,9-dien-11β-yl]benzaidehyde-1E-oxime and
4-{17β-Hydroxy-17α-(1,1,'1-trifluororoethoxymethyl)-3-oxoestra-4,9-dien-11β-yl]benzaldehyde-1E-oxime.

7. Compounds of formula (I), wherein R₁ is a CₙF₂ₙ₊₁ radical, whereby n is 1, 2 or 3, and R₂, X and Y have the meanings that are indicated in claim 1.

8. Compounds of formula (I), wherein Y is an OCH₂CₙF₂ₙ₊₁ group, whereby n is 1, 2 or 3, and R₁, R₂ and X have the meanings that are indicated in claim 1.

## Revendications

1. Procédé de préparation de 4-(17α-méthyl-substitués 3-oxoestra-4,9-dién-11β-yl)benzaldéhyde-(1E ou 1Z)-oximes de formule générale (I) 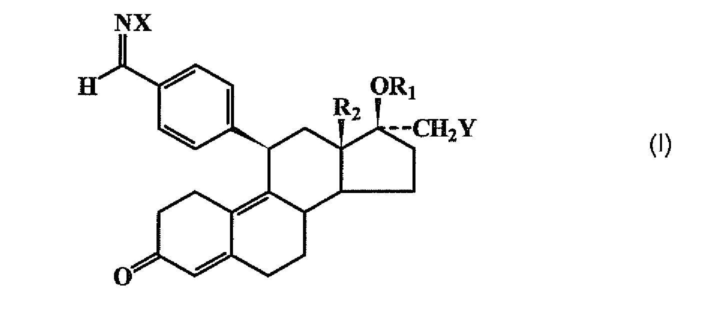 dans laquelle R₁ est un atome d'hydrogène, un radical alkyle en C₁₋₆ ou un radical CₙF_{2n+1,} n étant égal à 1, 2 ou 3, R₂ est un radical alkyle en C₁₋₄ ou un groupe trifluorométhyl, X est un groupe OH en configuration E ou Z et Y est un groupe OC₁₋₆-alkyle, SC₁₋₆-alkyle ou OCH₂CₙF₂ₙ₊₁, n étant égal à 1, 2 ou 3, **caractérisé en ce qu'**on transforme le 3,3-diméthoxy-estra-5(10),9(11)-dién-17-one de formule (IX) 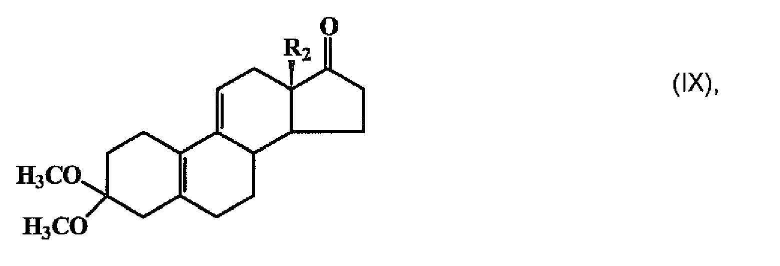 dans laquelle R₂ a la signification indiquée précédemment, avec un réactif méthylène actif dans un solvant inerte, en le spiroépoxyde de formule (X) 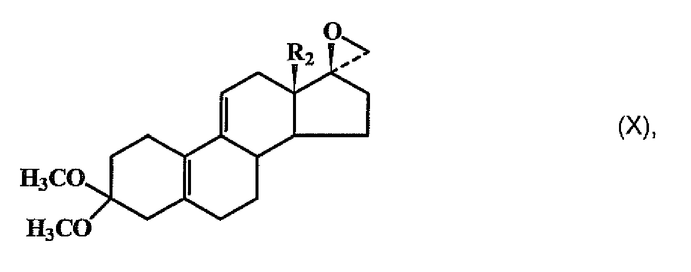 dans laquelle R₂ a la signification indiquée précédemment, et, après dissociation du groupe 17-spiroépoxy par un alcoolate alcalin ou alcalino-terreux, un thioalcoolate alcalin ou alcalino-terreux ou par des trifluoroalkyl-alcools et du tert-butanolate de potassium dans un solvant inerte, on l'ouvre pour former le composé 17α-CH₂Y de formule (XI) 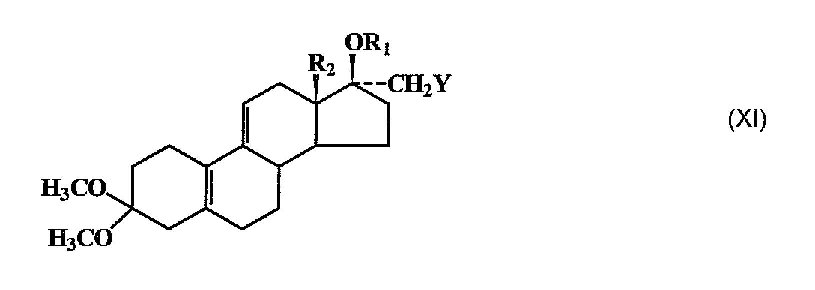 dans laquelle R₁ représente un atome d'hydrogène et R₂ et Y ont la signification mentionnée précédemment, et on transforme éventuellement ce composé par réaction du groupe 17β-hydroxyl avec des alkylhalogénures ou du fluoroalkyliodure en présence de bases fortes dans un solvant inerte pour obtenir les 17β-éthers de formule (XI), dans laquelle R₁ représente un radical alkyl en C₁₋₆ ou un radical CₙF₂ₙ₊₁ et R₂ et Y ont la signification indiquée précédemment, on époxyde le composé de formule (XI) à la double liaison 5(10), moyennant quoi il se forme un mélange de 5α,10α-époxy- et de 5β,10β-époxy-17α-méthyl-substitués estr-9(11)-éne-3,3-diméthylcétal de formule (XII) 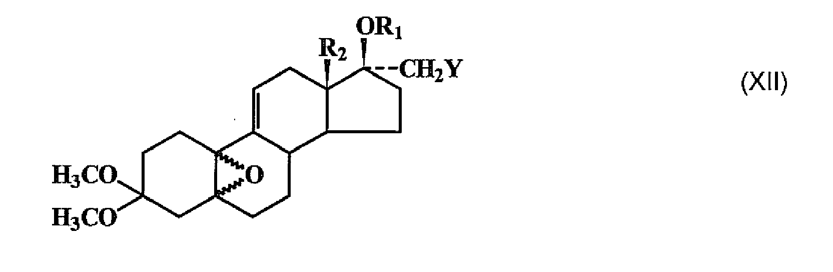 dans laquelle R₁, R₂ et Y ont la signification mentionnée précédemment, on ouvre le produit obtenu avec un 4-bromobenzaldéhydecétal, du magnésium et du Cu(I)Cl à des températures entre -35°C et la température ambiante pour former le 3,3-diméthoxy-5α-hydroxy-17α-méthyl-substitué 11α,β-benzaldéhydediméthyl-cétal correspondant de formule (XIII) 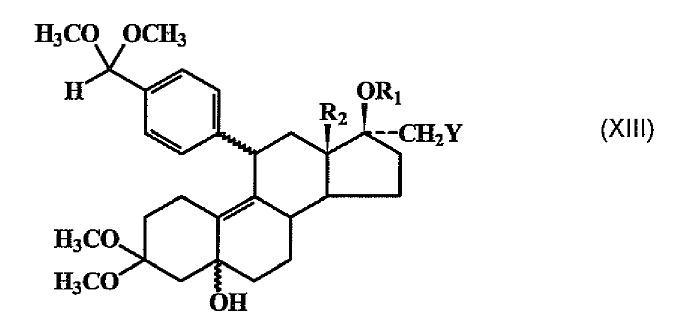 dans laquelle R₁, R₂ et Y ont la signification mentionnée précédemment, et on soumet ce dernier à une hydrolyse acide pour libérer les groupes protecteurs, moyennant quoi il se forme un mélange de dérivés de 11α,β-benzaldéhyde de formule (XIV) 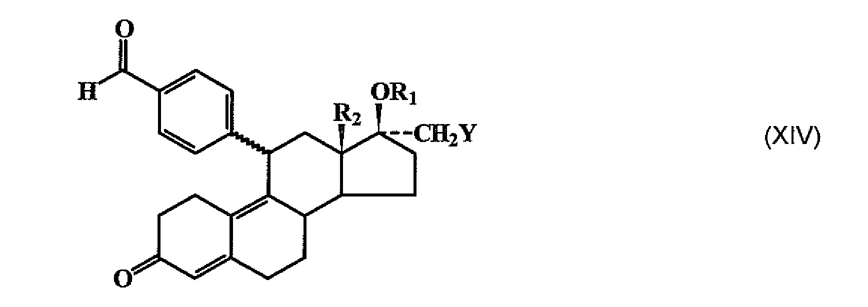 dans laquelle R₁, R₂ et Y ont la signification mentionnée précédemment, à partir duquel on isole par cristallisation les 11β-benzaldéhydes purs de formule (VII), dans laquelle R₁, R₂ et Y ont la signification mentionnée précédemment, et on transforme la fonction aldéhyde par des sels d'hydroxylammonium en présence de bases, de préférence de pyridine, à température ambiante pour former un mélange de E/Z-benzaldoximes de formule générale (1).

2. Procédé selon la revendication 1, **caractérisé en ce qu'**on sépare le mélange des E/Z-benzaldoximes de formule générale (1) par recristallisation et/ou par chromatographie pour obtenir les composants individuels.

3. Procédé selon l'une des revendications 1 ou 2, **caractérisé en ce que** R₁ représente un radical alkyle en C₁₋₃, de préférence un groupe méthyle ou un groupe trifluorométhyl.

4. Procédé selon l'une des revendications 1 à 3, **caractérisé en ce que** R₂ représente un radical alkyle en C₁₋₃, de préférence un groupe méthyle.

5. Procédé selon l'une des revendications 1 à 4, **caractérisé en ce que** Y représente un radical OC₁₋₃-alkyl ou SC₁₋₃-alkyl, de préférence un groupe méthoxy, éthoxy, isopropyloxy, méthylthio ou éthylthio, ou trifluoroéthoxy.

6. Procédé selon l'une des revendications 3 à 5, **caractérisé en ce que** l'on prépare les composés suivants :
4-[17β-hydroxy-17α-(méthoxyméthyl)-3-oxoestra-4,9-dién-11β-yl]benzaldéhyde-1E-oxime,
4-[17β-hydroxy-17α-(mëthoxyméthyl)-3-oxoestra-4,9-dién-11β-yl]benzaldéhyde-1Z-oxime,
4-[17β-méthoxy-17α-(méthoxyméthyl)-3-oxoestra-4,9-dién-11β-yl]benzaldëhyde-1E-oxime,
4-[17β-méthoxy-17α-(méthoxyméthyl)-3-oxoestra-4,9-dién-11β-yl]benzaldéhyde-1Z-oxime,
4-[17β-éthoxy-(17α-(méthoxyméthyl)-3-oxoestra-4,9-dién-11β-yl]benzaldéhyde-1E-oxime,
4-[17β-hydroxy-17α-(éthoxyméthyl)-3-oxoestra-4,9-dién-11β-yl]benzaldéhyde-1E-oxime,
4-[17β-mëthoxy-17α-(éthoxyméthyl)-3-oxoestra-4,9-dién-11β-yl]benzaldéhyde-1E-oxime,
4-[17β-hydroxy-17α-(isopropyloxyméthyl)-3-oxoestra-4,9-dién-11β-yl]benzaldéhyde-1E-oxime,
4-[17β-méthoxy-17α-(isopropyloxyméthyl)-3-oxoestra-4,9-dién-11β-yl]benzaldéhyde-1E-oxime,
4-[17β-hydroxy-17α-(éthylthiométhyl)-3-oxoestra-4,9-dién-11β-yl]benzaldéhyde-1E-oxime et
4-[17β-hydroxy-17α-(1,1,1-trifluoroéthoxyméthyl)-3-oxoestra-4,9-dién-11β-yl]benzaldéhyde-1E-oxime.

7. Composés de formule (I), **caractérisés en ce que** R₁ est un radical CₙF₂ₙ₊₁, n étant égal à 1, 2 ou 3, et R₂, X et Y ont la signification mentionnée dans la revendication 1.

8. Composés de formule (I), **caractérisés en ce que** Y est un groupe OCH₂CₙF₂ₙ₊₁, n étant égal à 1, 2 ou 3, et R₁, R₂ et X ont la signification indiquée dans la revendication 1.
